(19) Europäisches Patentamt
European Patent Office
Office européen des brevets

(11) **EP 0 986 356 B1**

(12) **EUROPÄISCHE PATENTSCHRIFT**

(45) Veröffentlichungstag und Bekanntmachung des Hinweises auf die Patenterteilung:
**11.07.2001 Patentblatt 2001/28**

(21) Anmeldenummer: **98913506.6**

(22) Anmeldetag: **23.04.1998**

(51) Int Cl.⁷: $A61F\ 13/08$, $A61H\ 23/04$

(86) Internationale Anmeldenummer:
**PCT/CH98/00162**

(87) Internationale Veröffentlichungsnummer:
**WO 99/53876 (28.10.1999 Gazette 1999/43)**

(54) **ORTHOSTASE-ANZUG**

SUIT FOR PROBLEMS ASSOCIATED WITH ORTHOSTASIS

TENUE VESTIMENTAIRE POUR PROBLEMES D'ORTHOSTASE

(84) Benannte Vertragsstaaten:
**AT BE CH DE DK ES FR GB IT LI NL PT SE**

(30) Priorität: **20.04.1998 CH 90298**

(43) Veröffentlichungstag der Anmeldung:
**22.03.2000 Patentblatt 2000/12**

(73) Patentinhaber: **Prospective Concepts AG**
**8702 Zollikon (CH)**

(72) Erfinder: **REINHARD, Andreas**
**CH-8702 Zollikon (CH)**

(74) Vertreter: **Salgo, Reinhold Caspar, Dr.**
**Rütistrasse 103**
**8636 Wald (CH)**

(56) Entgegenhaltungen:
**EP-A- 0 305 284**            **WO-A-95/24881**
**NL-A- 8 201 189**           **US-A- 4 502 470**
**US-A- 5 546 955**

**Beschreibung**

Die vorliegende Erfindung bezieht sich auf ein Kleidungsstück für Patienten mit Orthostase-Problemen nach dem Oberbegriff des Patentanspruches 1.

Bei Patienten mit Orthostase-Syndrom verschiedenster Genese versackt beim Uebergang vom Liegen zum Stehen das Blut im Bereiche der Bein- und Eingeweidevenen.

Um diese Erscheinung zu verhindern oder mindestens zu mildern sind sog. Stütz-Strümpfe oder -Hosen bekannt, die elastische Fäden eingewirkt enthalten und unter deren Einfluss einen Druck auf mindestens die Beine ausüben. Die Nachteile dieser elastischen Stütz-Strümpfe oder -Hosen liegen darin, dass sie auf die Beine bzw. den Unterleib auch einen Druck ausüben, wenn der Patient liegt, mühsam und oft nur mit Dritthilfe anzuziehen sind und überdies nur in fixen Grössen angeboten werden. Dies lässt weder Raum für individuelle Körpermasse ausserhalb der erhältlichen Grössen, noch für eine Anpassung an momentane Körperbedingungen.

Ein solcher Strumpf für den Unterschenkel ist aus NL 8201189 (D1) bekannt. Das verwendete Druckmedium ist dabei Luft, also ein Gasgemisch, so dass in jeder Höhe des Unterschenkels der gleiche Druck ausgeübt wird. Um mit dieser Vorrichtung dennoch verschiedene Drücke auf verschiedenen Höhen realisieren zu können, werden auf verschiedenen Höhen angeordnete getrennte Kompartimente vorgeschlagen, die einzeln manuell mit Druckluft beaufschlagt werden können.

Die Handhabung dieser Vorrichtung wird dadurch sehr umständlich, da für jede Lage des Unterschenkels diese Drücke wieder neu optimiert werden müssten; die Handhabung würde noch komplizierter und zeitraubender, wenn auch für den Oberschenkel oder gar für den Abdominalbereich weitere solcher Kompartimente eingesetzt würden.

Ein anderer solcher Strumpf ist aus US 5,546,955 (D2) bekannt. Der Unterschenkel wird dabei von verschiedenen, längs nebeneinander oder vertikal übereinander angeordneten Kompartimenten oder Druckkammern umfasst, von denen alle einzeln über elektronisch gesteuerte Ventile in vorwählbaren Zeitintervallen rythmisch mit Druck einer Flüssigkeit aus einem Reservoir beaufschlagt werden können. Temperatursensoren überwachen die Temperatur des Unterschenkels. Bei Unterkühlung des Unterschenkels kann die Flüssigkeit beheizt werden.

Diese Vorrichtung dient - unabhängig von der Lage des Unterschenkels - der Unterstützung des Blutkreislaufs und gleichzeitig der Erwärmung des Unterschenkels, was wegen der grossen Wärmekapazität von Flüssigkeiten gegenüber Gasen sozusagen zwangsläufig zur Wahl einer Flüssigkeit als Druckmedium führt. Die Vorrichtung ist, wegen der vorhandenen elektronischen Mess- und Steuervorrichtungen, noch umständlicher zu bedienen als der aus D1 bekannte manuell zu bedienende Strumpf; eine Uebertragung des vorgeschlagenen Prinzips auf Oberschenkel und Abdominalbereich ist, unter anderem wegen der grossen Flüssigkeitsmassen, die dazu nötig wären, praktisch ausgeschlossen.

Die Aufgabe der vorliegenden Erfindung ist es, einen Orthostase-Anzug zu schaffen, der sowohl auf cas individuelle Körpermass als auch auf dessen Momentanwert eingestellt werden kann. Ferner soll der erfindungsgemässe Anzug auf die Stärke des vorliegenden Orthostase-Syndroms eingestellt werden können.

Die Lösung der gestellten Aufgabe ist in ihren wesentlichen Merkmalen wiedergegeben im kennzeichnenden Teil des Patentanspruchs 1, in weiteren vorteilhaften Merkmalen in den davon abhängigen Ansprüchen.

Anhand der beigefügten Zeichnungen ist der Erfindungsgedanke anhand von Ausführungsbeispielen näher beschrieben. Es zeigen

Fig. 1        einen Querschnitt durch den Schichtaufbau des Orthostase-Anzugs,

Fig. 2a        eine Draufsicht,

Fig. 2b        einen ersten Schnitt,

Fig. 2c        einen zweiten Schnitt,

Fig. 2d        einen dritten Schnitt durch eine erste Anordnung von Verbindungsstellen,

Fig. 3        einen ersten Querschnitt durch einen Teil eines Orthostase-Anzugs,

Fig. 4        eine zweite Anordnung von Verbindungsstellen,

Fig. 5        eine dritte Anordnung von Verbindungsstellen,

Fig. 6        eine Frontansicht auf ein erstes Ausführungsbeispiel eines Orthostase-Anzugs,

Fig. 7        einen zweiten Querschnitt durch einen Teil eines Orthostase-Anzugs mit Schliessvorrichtung,

Fig. 8        der Gegenstand von Fig. 7 mit einer Spannvorrichtung,

Fig. 9        die Ansicht einer erfindungsgemässen Ergänzung,

Fig. 10        eine Frontansicht auf ein zweites Ausführungsbeispiel eines Orthostase-Anzugs,

Fig. 11        eine Frontansicht auf ein drittes Ausführungsbeispiel eines Orthostase-Anzugs.

Der erfindungsgemässe Orthostase-Anzug - bestehend aus zwei strumpfähnlichen Elementen - oder ausgebildet als Hose - ist grundsätzlich aufgebaut aus zwei unterschiedlichen textilen Komponenten: Die erste Komponente ist ein wenig dehnbares textiles, dampfdurchlässiges Material, beispielsweise gefertigt aus Aramidfasern; die zweite Komponente, ebenfalls textiler Natur, besteht aus zwei Schichten eines flüssigkeitsdichten Materials. Dabei sind die beiden Schichten miteinander stellenweise verbunden durch Schweissen, Kleben oder Nähen mit anschliessendem Abdichten.

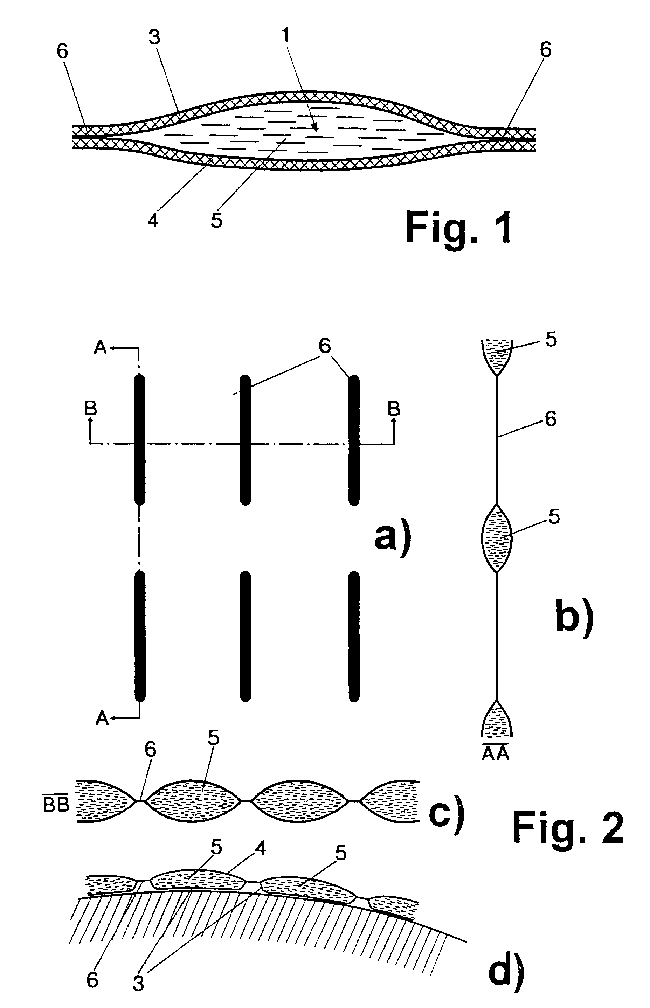

Fig. 1 zeigt einen Querschnitt durch diese zweite Komponente: Sie besteht aus einer inneren - also körpernahen - flüssigkeitsdichten textilen Schicht 3 und einer aus dem gleichen Material bestehenden äusseren Schicht 4. Die Schichten 3, 4 sind an Verbindungsstellen 6 durch Kleben, Schweissen oder Nähen (mit einer dichten oder abgedichteten Naht) miteinander verbunden. Dadurch entsteht zwischen den Schichten 3, 4 ein Hohlraum 5, der mit einer Flüssigkeit 1 gefüllt werden kann. Die Flüssigkeit 1 kann aus Wasser bestehen, das mittels bakteriostatischer Zusätze stabilisiert ist und allenfalls weitere Zusätze zur Einstellung der Dichte und der Fliessfähigkeit enthält.

Fig. 2 a, b, c, d, zeigt in Detailansichten das Anbringen von Verbindungsstellen 6 der Schichten 3, 4. Wie bereits erläutert, können diese Verbindungsstellen durch Schweissen, Kleben oder Nähen hergestellt werden. In Fig. 2a ist schematisch aus einem Teil des Schutzanzuges ein Feld von beispielsweise sechs Verbindungsstellen 6 dargestellt. Jede einzelne Verbindungsstelle hat die Form eines langen schlanken Streifens. Ein Schnitt AA gemäss Fig. 2b zeigt, dass der Abstand zwischen den Enden der streifenförmigen Verbindungsstellen 6 verkürzt wird, sobald die im Hohlraum 5 zwischen den Schichten 3, 4 befindliche Flüssigkeit zuströmt und unter Druck gesetzt wird. Dasselbe gilt für den seitlichen Abstand der Verbindungsstellen 6, wie im Schnitt BB gemäss Fig. 2c gezeigt.

Wird nun ein aus den Schichten 3, 4 bestehendes Gebilde um beispielsweise einen Oberschenkel herumgelegt, so ergibt sich, was in Fig. 2d schematisch dargestellt ist:

Die äussere Schicht 4 wird gespannt auf eine Zugspannung σ, die innere Schicht 3 legt sich - im wesentlichen spannungslos - an die Körperoberfläche an; im Inneren der Hohlräume 5 herrscht der Druck p. Dieser baut die Zugspannung σ auf, die über die Verbindungsstellen 6 übertragen wird, so dass ein bestimmter Druck p einer bestimmten Zugspannung entspricht.

Werden nun zwei - im Schnitt dargestellte - Hohlräume 5 so angeordnet, dass zwischen Ihnen eine Trennzone 7 liegt, die keine Hohlräume 5 enthält, so wird die Zugspannung σ im wesentlichen ohne Abbau von Hohlraum 5 zu Hohlraum 5 weitergegeben. Der Abbau der Zugspannung, der normalerweise mit einem

Umschlingungswinkel α einhergeht:

$$\sigma(\alpha)=\sigma_0 \cdot e^{-\alpha \cdot f_H}$$

wo

$\sigma_0$ = Einleitungsspannung
$f_H$ = Haftreibungsbeiwert,

gilt nur für starre umschlungene Körper. Menschliches Körpergewebe ist jedoch weitgehend nachgiebig und verformbar.

Die Trennzone 7 wird vorzugsweise aus der ersten Komponente, also einem dampfdurchlässigen textilen Material kleiner Dehnbarkeit gefertigt.

Die Verbindungsstellen 6 sind den Hohlräumen unmittelbar benachbart. Sie können, wie in Fig. 1, 2 gezeigt, die Schichten 3, 4 verbinden, oder zusätzlich die Verbindung zu dem textilen Material sicherstellen, aus welchem die Trennzone 7 hergestellt ist.

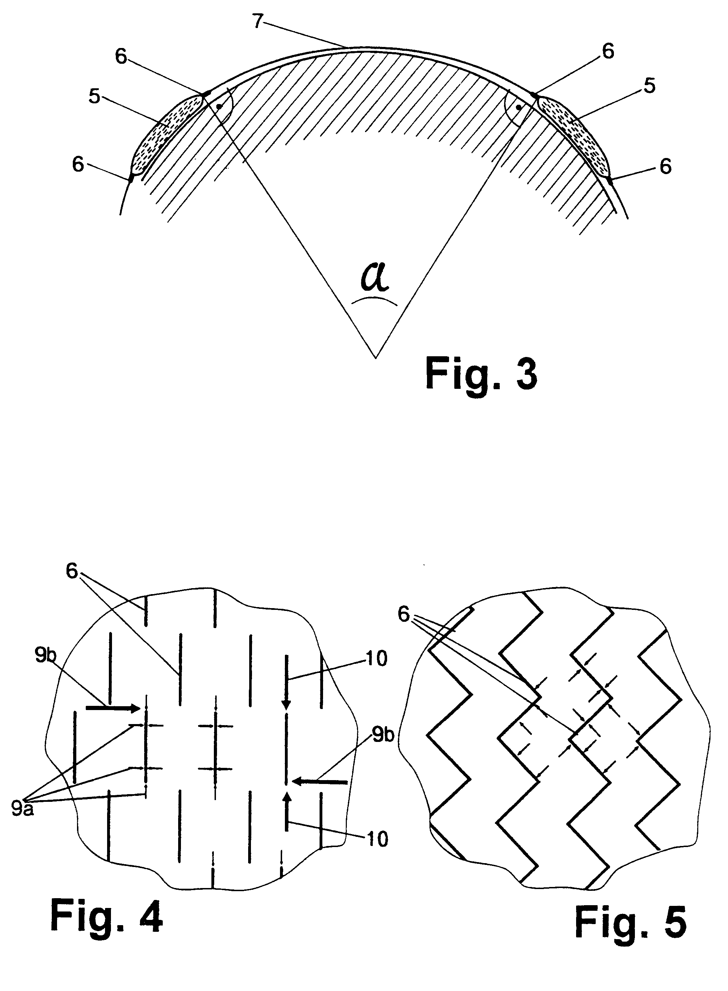

In Fig. 4 sind die linear ausgebildeten Verbindungsstellen in gegeneinander versetzte Reihen angeordnet. Durch Druckanwendung auf die Flüssigkeit, die sich in den zwischen den Schichten 3, 4 entstehenden Hohlräumen befindet, entstehen Kraftwirkungen auf die Verbindungsstellen 6 (kleine Pfeile 9a in Fig. 4). Dadurch verkürzt sich der aus den Schichten 3, 4 bestehende Aufbau vorzugsweise in der Richtung quer zu der Richtung der Verbindungsstellen 6 (grosse Pfeile 9b in Fig. 4). In weniger starkem Masse entsteht durch diese Anordnung jedoch auch eine solche Verkürzung in der Richtung der linearen Verbindungsstellen 6 (grosse Pfeile 10). Die daraus entstehenden Zugspannungen σ verhalten sich dann in derselben Ordnung, so dass $\sigma_{quer} > \sigma_{längs}$. Die zwischen den Schichten 3, 4 befindliche Flüssigkeit hat bei dieser Anordnung grosse Mobilität; sie kann sowohl längs als auch quer zur Richtung der linearen Verbindungsstellen 6 strömen.

Die Anordnung gemäss Fig. 5 baut gegenüber jener von Fig. 4 annähernd isotrope Zugspannungen auf, da durch das Zickzack-Muster der Verbindungsstellen 6 die Projektionen in beiden Koordinatenrichtungen der Ebene der Schichten 3, 4 annähernd gleich gross sind, oder doch mindestens gleich gross sein können. Damit ist auch ein annähernd isotropes Schrumpfen der Grösse des so mit Verbindungsstellen 6 versehenen Flächenstückes gegeben.

Die Beweglichkeit der Flüssigkeit in den Hohlräumen 5 zwischen den Verbindungsstellen 6 gemäss Fig. 5 ist durch das vorgegebene Muster insofern eingeschränkt, als sie nicht quer zu den Verbindungsstellen 6 möglich ist. Anstelle des dargestellten Zickzackmusters mit scharfen Ecken ist auch eine Ausbildung mit Rundungen im Erfindungsgedanken mitenthalten; anstelle eines Zickzackmusters im engeren Sinne tritt dann ein wellenförmiges. Unter diesem Begriffe sind alle solchen Ausbildungen zusammengefasst und zu verstehen.

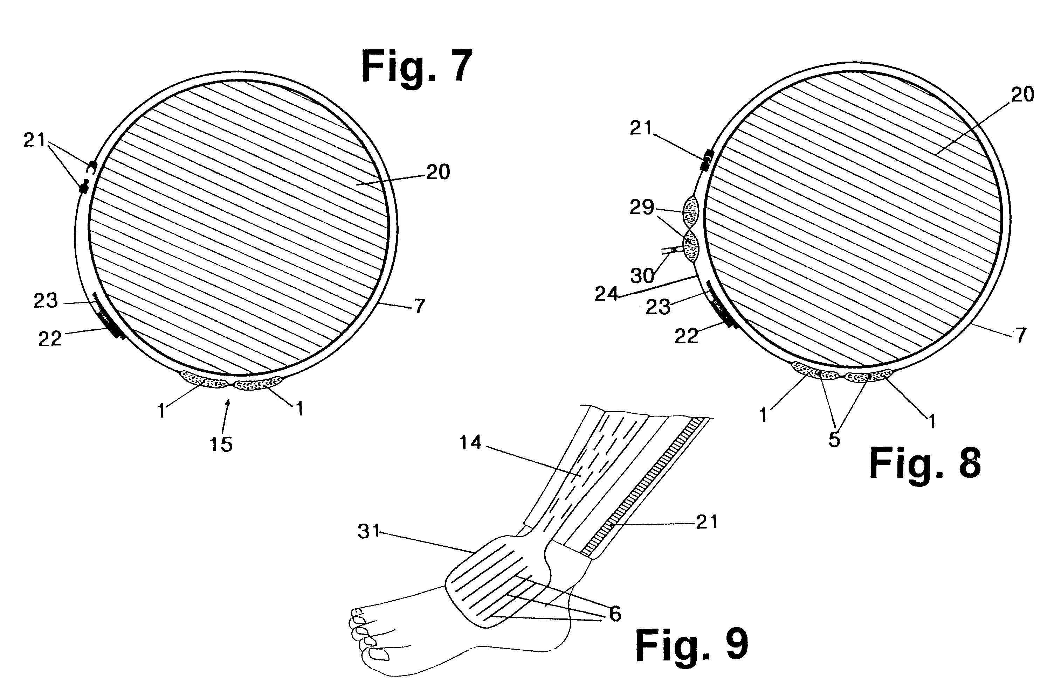

Fig. 6 zeigt den erfindungsgemässen Orthostase-Anzug an einem ersten Ausführungsbeispiel mit Varianten hinsichtlich der Anordnungen der Verbindungsstellen 6, dargestellt als zwei Bildhälften "links" und "rechts".

Das Ausführungsbeispiel gemäss Fig. 6 ist als Hose ausgeführt mit Hosenträgern 11, die zugleich als Reservoirs 12 für die Flüssigkeit 1 ausgebildet sind. Die Flüssigkeitssäule erzeugt in den Hohlräumen den Schweredruck

$$p = \rho gh$$

wo

p = Druck

$\rho$ = Dichte der Flüssigkeit

h = Höhendifferenz vom oberen Flüssigkeitsspiegel bis zur betrachteten Stelle im Orthostase-Anzug

Unterhalb der beiden Reservoirs 12 befindet sich eine flache Blase 13 (gestrichelt eingezeichnet), die sich innerhalb des dehnungsarmen textilen Materials der ersten Komponente befindet, welches hier die Bauch/Abdominalregion abdeckt. In die Blase 13 münden mindestens an ihrem oberen Rand von unten mindestens zwei Streifen 14, 15, die je eine Variante eines Musters von Verbindungsstellen 6 tragen. Der Streifen 14, links trägt ein Muster gemäss Fig. 4, der Streifen 15, rechts eines gemäss Fig. 5. Selbstverständlich werden bei einem bestimmten Ausführungsmuster die beiden Streifen 14, 15 gleich gestaltet sein; sie erstrecken sich von der Blase 13 bis oberhalb des Fussknöchels. Nicht dargestellt, jedoch im Erfindungsgedanken enthalten sind nach oben oder/und nach unten verzweigte Bänder. Damit können der Spannungsverlauf und die anatomische Anpassung optimiert werden.

Je nach dem Füllgrad der Reservoirs 12 und der Dichte p der Flüssigkeit wird also im Stehen ein Kompensationsdruck aufgebaut, der im wesentlichen der Blutdruckdifferenz über die entsprechende Höhendifferenz entspricht. Der Kompensationsdruck wird durch die Ausgestaltung der Muster der Verbindungsstellen 6 in eine Umlaufspannung σ umgesetzt, die in den Trennzonen 7 herrscht; diese wiederum baut rund um die vom Orthostase-Anzug eingeschlossenen Körperteile den Kompensationsdruck auf.

Die Region der Kniescheibe ist - um die normale Beweglichkeit zu erhalten - von der Zugspannung ausgenommen; dort ist je ein elastisches textiles Material in Form von runden oder länglichen Einsätzen 16 vorgesehen. Die Genitalregion ist ebenso wie die Knie von der Zugspannung ausgenommen. Entweder bleibt sie vom Orthostase-Anzug unbedeckt oder wird ebenfalls von einem elastischen Einsatz 17 abgedeckt.

Alternativ kann die ganze Knieregion im wesentlichen vom Anzug unbedeckt bleiben; die Verbindung von Oberschenkelteil und Unterschenkelteil wird dann durch einen vorzugsweise lateral liegenden Streifen textilen Materials, das den Streifen 14 oder 15 enthält, gewährleistet.

In Fig. 6 ist je ein Streifen 14, 15 eingetragen. Selbstverständlich ist es im Erfindungsgedanken eingeschlossen, mehr als nur einen Streifen vorzusehen. Einerseits kann dadurch der Umfang des Orthostase-Anzugs stärker verkürzt werden, was die Beweglichkeit etwas erhöht, anderseits ist dann die notwendige Flüssigkeitsmenge - und damit das Gewicht des Orthostase-Anzugs auch entsprechend höher.

Fig. 7 zeigt die Schliessvorrichtung des Orthostase-Anzugs gemäss Fig. 6. Um ein schematisch dargestelltes Bein 20 ist der vor allem aus der Trennzone 7 bestehende Crthostase-Anzug herumgelegt. Der Streifen 15 (oder 14) wird diese in stehender Stellung des Trägers durch Druckaufbau in der Flüssigkeit 1 spannen.

Um dem Träger des erfindungsgemässen Orthostase-Anzuges grösstmögliche Selbständigkeit zu erhalten und die richtige Funktion des Orthostase-Anzugs zu gewährleisten, ist die Schliessvorrichtung zweiteilig: Ein Klettverschluss 22, 23 dient zur Anpassung des Orthostase-Anzugs an die momentane Körpersituation des Trägers. Ein Reissverschluss 21 dient, nach erfolgter Anpassung, zum Schliessen des Orthostase-Anzugs in liegender Stellung des Trägers. Während für die optimale Anpassung Dritt-Hilfe notwendig ist, kann das Schliessen des Reissverschlusses 21 durch den Träger allein erfolgen. Dazu kann der Reissverschluss 21 so eingebaut werden, dass er von oben nach unten schliesst; das Zusammenstecken der beiden Teile des Reissverschlusses 21 kann dann oben erfolgen, das Schliessen notfalls mit Hilfe eines Stabes, der in die Schliessvorrichtung des Reissverschlusses 21 eingesetzt wird. Klettverschluss 22, 23 und Reissverschluss 21 sind sowohl in Fig. 6 als auch in Fig. 7 aussenliegend und nahe benachbart gezeichnet. Selbstverständlich können die Klettverschlüsse 22, 23 an den Beinen proximal und die Reissverschlüsse 21 lateral angeordnet sein. Erfindungswesentlich ist das Vorhandensein einer Vorrichtung zum Anpassen (also beispielsweise der Klettverschluss 22, 23) und eine Vorrichtung zum Schliessen (also der Reissverschluss 21). Deren räumliche Anordnung soll so vorgenommen werden, um dem Träger des Orthostase-Anzugs grösstmöglichen Komfort sowohl beim An- und Ausziehen, als auch beim Tragen des Orthostase-Anzugs zu bieten.

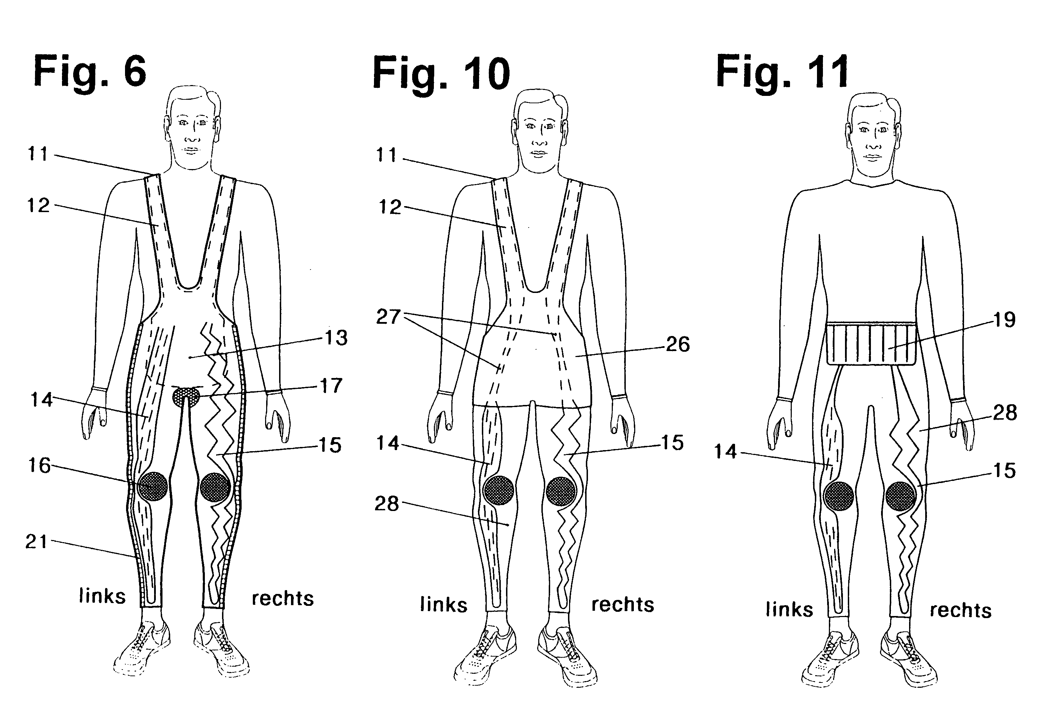

Fig. 8 ist die Darstellung einer weiteren erfindungsgemässen Ergänzung. Ein oder mehrere luftdicht und beispielsweise wie die Streifen 14 aufgebaute Kanäle 29 ziehen sich über die ganze Länge des Orthostase Anzugs gemäss Fig. 6 oder über diejenige des Beinteils gemäss Fig. 11. Die Kanäle 29 sind beispielsweise angeordnet am Lappen 31 zwischen Reissverschluss 21 und Klettverschluss 22, 23. Nach dem Schliessen von Klettverschluss 22, 23 und Reissverschluss 21 kann der Träger des Orthostase Anzuges eine handbetätigte Luftpumpe an einem Ventil 30 ansetzen und die Kanäle 29 damit mit Luftdruck beaufschlagen. Dadurch wird ein dichter Sitz des Orthostase Anzuges gewährleistet und der Reissverschluss 21 lässt sich leichter schliessen.

Oft sind die in den Füssen verlaufenden Venen vom Orthostase-Syndrom mitbetroffen. In diesen Fällen kann es hilfreich sein, den Fussbereich in den Aufbau des Kompensationsdruckes miteinzubeziehen. Fig. 9 zeigt - als Detail - diesen Einbezug.

An den Streifen 14 oder 15 schliesst sich ein Lappen 31 an, der beispielsweise parallele Verbindungsstellen 6 trägt. Dieser Lappen wird in den vorzugsweise hohen oder halbhohen Schuh unter die Lasche eingelegt. Voraussetzung ist natürlich ein über den Rist wenig dehnbarer Schuh. Damit wird durch das Wirken des Kompensationsdruckes auch im Schuh die notwendige Umlaufspannung aufgebaut. Der Lappen 31 kann in seinem Zuschnitt auf die anatomischen Gegebenheiten des Fusses angepasst werden.

Ist die Bauch/Abdominalregion vom Orthostase-Syndrom nicht oder wenig betroffen, so kann dieser Teil entweder weggelassen oder aber vorzugsweise wesentlich anspruchsloser gestaltet sein. Fig. 10 zeigt dieses Ausführungsbeispiel.

Von den in die Hosenträger 11 integrierten Reservoirs 12 gelangt die Flüssigkeit 1 durch zwei verhältnismässig dünn ausführbare Kanäle 27 in die Streifen 14, bzw. 15.

Während die Ausgestaltung des Beinteiles des Orthostase-Anzugs gleich ist, wie jene gemäss Fig. 6, also aus wenig dehnbarem Textilmaterial, kann der obere aus gängigen, vorzugsweise synthetischen, Textilien ausgeführt sein. Dieser obere Teil 26 hat die Aufgabe, die Beinteile 28 zu halten, ist Kraftangriff für die Hosenträger 11 und hält die Kanäle 27 an der richtigen Stelle. Der Füllgrad der Reservoirs hängt wiederum ab von der Anzahl der Streifen 14, bzw. 15. Auch dieses Ausführungsbeispiel kann ergänzt werden durch je einen Lappen 31 gemäss Fig. 9. Anstelle des in Fig. 6 dargestellten in die Hosenträger 11 integrierten Reservoirs 12 ist auch ein - nicht dargestelltes - westenartiges Oberteil erfindungsgemäss, das Träger ist für ein Reservoir 19, das sowohl die Streifen 14 bzw. 15, als auch die Blase 13 speist.

Kann der Kompensationsdruck aufgrund der medizinischen Indikation klein gehalten werden, so besteht die Möglichkeit auf die Hosenträger 11 zu verzichten. Die Reservoirs 12 werden dann, wie in Fig, 11 gezeigt, zum einzigen, vorzugsweise segmentierten Reservoir 19 zusammengefasst, das sich in der Bauchregion befindet. Damit reduziert sich der maximale Kompensationsdruck unter Berücksichtigung der Dichte $\rho$ der Flüssigkeit 1 auf die Höhendifferenz zwischen Reservoir 19 und der tiefsten Stelle des Beinteils 28.

Das Reservoir 19 ist mit den Streifen 14 bzw.

15 wiederum durch Kanäle 27 verbunden. Auch hier ist die Ergänzung durch Lappen 31 erfindungsgemäss.

Ein weiterer erfindungsgemässer Zusatz besteht darin, sowohl Oberteil 26 als auch Beinteile 28 teilweise oder über deren ganze Länge mit einem elastischen Einsatz zu ergänzen, der die mechanische Charakteristik einer sogenannten zurückgewundenen Feder aufweist, also erst ab einer bestimmten, wählbaren Umlaufspannung $\sigma$ elastisch reagiert. Diese elastischen Einsätze verlaufen kräftemässig parallel zu dem Reissverschluss 21 und sind beispielsweise zwischen Reissverschluss 21 und Klettverschluss 22, 23 oder aber entlang der proximalen Meridiane der Beinteile 28 vorzusehen.

## Patentansprüche

1. Orthostase-Anzug zur Kompensation von verminderter oder fehlender Fähigkeit von Patienten zur Regelung des Blutdruckes im hypotonischen Bereich, insbesondere beim Wechsel von liegender zu stehender Stellung, mit längs der vom Orthostase-Anzug bedeckten Körperteile eingearbeiteten doppelwandigen, in mindestens zwei vertikalen Streifen (14, 15) angeordneten Gebieten, die aus zwei Schichten (3, 4) eines flüssigkeitsdichten, wenig dehnbaren textilen Materials bestehen, welche an Verbindungsstellen (6) miteinander verbunden sind und dazwischen Hohlräume (5) bilden, die mit einer Flüssigkeit (1) gefüllt werden können, wobei er neben den vertikalen Streifen (14, 15) Trennzonen (7) enthält, mit Mitteln, um den Orthostase-Anzug längs der von ihm bedeckten Körperteile zu schliessen, mit Mitteln, um den Orthostase-Anzug an die gegenwärtigen Grössenbedingungen seines Trägers anzupassen, und mit Mitteln, um den Orthostase-Anzug zu spannen, dadurch gekennzeichnet, dass

   - der Orthostase-Anzug gleichzeitig sowohl mindestens einen Teil des Ober- als auch mindestens einen Teil des Unterschenkels bedeckt,
   - die Verbindungsstellen (6) in Mustern angeordnet sind und am Rand der Streifen (14, 15) die flüssigkeitsdichten Schichten (3, 4) mit dem dampfdurchlässigen Material der Trennzonen (7) verbinden,
   - beim Aufstehen des Trägers eine in den Hohlräumen (5) befindliche Flüssigkeit (1) durch ihren hydrostatischen Druck mindestens teilweise kompensatorisch auf den in den bedeckten Körperteilen herschenden Blutdruck wirken kann.

2. Orthostase-Anzug nach Patentanspruch 1, dadurch gekennzeichnet, dass ein Reservoir (12) für die Flüssigkeit (1) vorhanden ist und über dem die Hohlräume (5) aufweisenden Gebiete liegt.

3. Orthostase-Anzug nach Patentanspruch 1, dadurch gekennzeichnet, dass die Trennzonen (7) aus einem dampfdurchlässigen Material bestehen.

4. Orthostase-Anzug nach einem der Patentansprüche 1 bis 3, dadurch gekennzeichnet, dass

 - die Verbindungsstellen (6) gerade Strecken sind, die im Wesentlichen parallel zueinander verlaufen, deren Länge etwa deren seitliche Abständen entsprechen,
 - die einzelnen Verbindungsstellen (6) im Wesentlichen auf zwei Scharen von parallelen Linien liegen, die um etwa den halben seitlichen Abstand zweier benachbarter Verbindungsstellen (6) gegeneinander versetzt sind.

5. Orthostase-Anzug nach einem der Patentansprüche 1 bis 3, dadurch gekennzeichnet, dass die Hohlräume (5) führenden Gebiete in Streifen (14, 15) angeordnet sind, die sich über die ganze Länge des Orthostase-Anzuges erstrecken.

6. Orthostase-Anzug nach Patentanspruch 5, dadurch gekennzeichnet, dass die Streifen (14) im Wesentlichen geradlinig sind.

7. Orthostase-Anzug nach Patentanspruch 5, dadurch gekennzeichnet, dass die Streifen (15) zickzackförmig sind.

8. Orthostase-Anzug nach einem der Patentansprüche 1 bis 7, dadurch gekennzeichnet, dass er ein hosenartiges Oberteil (26) aufweist, welches zusätzlich mindestens einen Teil des Rumpfs bedeckt.

9. Orthostase-Anzug nach Patentanspruch 8, dadurch gekennzeichnet, dass

 - das hosenartige Oberteil (26) mindestens die Bauch/Abdominalregion bedeckt,
 - er eine flache Blase (13) aufweist, die innerhalb des Orthostase-Anzuges in der Bauch-/Abdominalregion angeordnet ist und mit dem Orthostase-Anzug verbunden sein kann.

10. Orthostase-Anzug nach Patentanspruch 8, dadurch gekennzeichnet, dass er einen Gürtel mit einem Reservoir (12) aufweist, das mit dem Orthostase-Anzug verbunden ist.

11. Orthostase-Anzug nach Patentanspruch 8, dadurch gekennzeichnet, dass das hosenartige Oberteil (26) Hosenträger (11) aufweist.

12. Orthostase-Anzug nach Patentanspruch 11, dadurch gekennzeichnet, dass ein Reservoir (12) in die Hosenträger (11) eingearbeitet ist.

13. Orthostase-Anzug nach einem der Patentansprüche 1 bis 12, dadurch gekennzeichnet, dass er längs verlaufende elastische Einsätze aufweist.

14. Orthostase-Anzug nach einem der Patentansprüche 1 bis 3, dadurch gekennzeichnet, dass die Mittel zum Schliessen aus je einem Reissverschluss (21) pro Bein bestehen.

15. Orthostase-Anzug nach einem der Patentansprüche 1 bis 3, dadurch gekennzeichnet, dass die Mittel zum Anpassen aus je einem Klettverschluss (22, 23) pro Bein bestehen, wobei der eine Teil des Klettverschlusses (22) an einem Lappen (31), der andere Teil (23) an dem den Körper umschliessenden Teil des Orthostase-Anzuges befestigt ist.

16. Orthostase-Anzug nach Patentansprüchen 14 und 15, dadurch gekennzeichnet, dass die Mittel zum Spannen aus längs des Orthostase-Anzuges und im Lappen (31) zwischen Reissverschluss (21) und Klettverschluss (22) angeordneten Kanälen (29) bestehen, die mit Druckluft gebläht werden können und damit eine dem vorgesehenen Mindestdruck entsprechende Umlaufspannung des Orthostase-Anzuges erzeugen können.

17. Orthostase-Anzug nach Patentanspruch 1, dadurch gekennzeichnet, dass am unteren Ende der Streifen (14, 15) ein Lappen (31) befestigt ist, welcher den gleichen Aufbau aufweist, wie die Streifen (14, 15), ebenfalls mit der gleichen Flüssigkeit (1) gefüllt ist, wie diese, und dass die Hohlräume (5) von Streifen (14, 15) und Lappen (31) kommunizieren, wobei der Lappen (31) als Lasche in den Schuh des Trägers gelegt und mit der normalen Schliesseinrichtung des Schuhs auf dem Fuss fixiert werden kann.

18. Orthostase-Anzug nach Patentanspruch 1, dadurch gekennzeichnet, dass die Streifen (14, 15) verzweigt sein können.

**Claims**

1. An orthostasis suit for the compensation of lowered or failing capability of patients to regulate the blood pressure in the hypotonic region, especially during the change from a lying to a standing position, with at least two double walled regions along the body parts covered by the orthostasis suit built in, arranged in vertical strips (14, 15), which comprise two layers (3, 4) of a fluid tight low stretch textile material, which are joined together at connecting positions (6) and form hollow spaces (5) between them, which can be filled with a fluid (1), whereby it includes separation zones (7) alongside the vertical strips (14, 15), with means for the closure of the orthostasis suit along the body parts covered by it, with means to fit the orthostasis suit to the present size conditions of its wearer, and with means to tension the orthostasis suit, characterised in that

- the orthostasis suit simultaneously covers both at least one part of the upper as well as at least one part of the lower leg,
- the connecting positions (6) are arranged in patterns and connect the fluid tight layers (3, 4) at the edge of the strips (14, 15) with the vapour permeable material of the separation zones (7),
- when the wearer stands up the fluid (1) present in the hollow spaces (5) can act in compensation on the blood pressure in the covered body parts owing to its hydrostatic pressure.

2. An orthostasis suit according to Claim 1, characterised in that a reservoir (12) for the fluid (1) is present and lies above the regions having hollow spaces (5).

3. An orthostasis suit according to Claim 1, characterised in that the separation zones (7) comprise a vapour permeable material.

4. An orthostasis suit according to one of the Claims 1 to 3, characterised in that

- the connecting positions (6) are in straight lines which run essentially parallel to each other, whose length corresponds somewhat to their lateral separation,
- the individual connecting positions (6) lie essentially on two sets of parallel lines, which are displaced by about half the lateral separation of two adjacent connecting positions (6).

5. An orthostasis suit according to one of the Claims 1 to 3, characterised in that the regions carrying hollow spaces (5) are arranged in strips (14, 15), which extend over the whole length of the orthostasis suit.

6. An orthostasis suit according to Claim 5, characterised in that the strips (14) are essentially straight.

7. An orthostasis suit according to Claim 5, characterised in that the strips (15) are zig-zag shaped.

8. An orthostasis suit according to one of the Claims 1 to 7, characterised in that it has a trousers-like outer part (26) which additionally covers at least a part of the trunk.

9. An orthostasis suit according to Claim 1 or Claim 2, characterised in that

- the trousers-like outer part covers at least the belly/abdominal region,
- it has a flat bladder (13), which is arranged inside the orthostasis suit in the belly/abdominal region and can be connected to the orthostasis suit.

10. An orthostasis suit according to Claim 8, characterised in that it has a belt with a reservoir (12), which is connected to the orthostasis suit.

11. An orthostasis suit according to Claim 8, characterised in that the trousers-like outer part (26) has braces (11).

12. An orthostasis suit according to Claim 11, characterised in that a reservoir (12) is worked into the braces (11).

13. An orthostasis suit according to one of the Claims 1 to 12, characterised in that it has elastic inserts running longitudinally.

14. An orthostasis suit according to one of the Claims 1 to 3, characterised in that the means for closure comprise a zip fastener (21) for each leg.

15. An orthostasis suit according to one of the Claims 1 to 3, characterised in that the means for matching comprise a Velcro fastener (22, 23) for each leg, whereby one part of the Velcro fastener (22) is fastened to a cloth (31), the other part (23) to the part of the orthostasis suit surrounding the body.

16. An orthostasis suit according to Claim 14 and Claim 15, characterised in that the means for tensioning comprise channels (29) arranged along the orthostasis suit and in the cloth (31) between the zip fastener (21) and the Velcro fastener (22), which can be inflated with compressed air and thereby generate a peripheral tension corresponding to the predetermined least pressure.

17. An orthostasis suit according to Claim 1, characterised in that at the lower ends of the strips (14, 15) a cloth (31) is fastened, which has the same construction as the strips (14, 15) and is similarly filled with the same fluid (1) as them and that the hollow spaces (5) of the strips (14, 15) and the cloth (31) communicate, so that the cloth (31) can be placed as a tongue in the shoe of the wearer and fixed on the foot using the normal closing device of the shoe.

18. An orthostasis suit according to Claim 1, characterised in that the strips (14, 15) can be branched.

## Revendications

1. Vêtement pour problèmes orthostatiques, destiné à compenser l'aptitude réduite ou insuffisante de patients à réguler la pression artérielle dans la région hypotonique, en particulier lors du passage de la position allongée à la position debout, comportant des zones à double paroi qui sont ménagées le long des parties du corps couvertes par ledit vêtement, qui sont disposées dans au moins deux bandes verticales (14, 15) et qui se composent de deux couches (3, 4) d'un matériau textile étanche au liquide et peu extensible, reliées entre elles au niveau de points de liaison (6) et formant entre elles des cavités (5) qui peuvent être remplies d'un liquide (1), étant précisé que le vêtement pour problèmes orthostatiques contient, près des bandes verticales (14, 15), des zones de séparation (7), et comportant des moyens pour fermer ledit vêtement le long des parties du corps qu'il couvre, des moyens pour adapter ledit vêtement aux conditions de taille présentes de la personne qui le porte, et des moyens pour tendre ledit vêtement,

    caractérisé en ce que

    - le vêtement pour problèmes orthostatiques couvre en même temps une partie au moins de la cuisse et une partie au moins de la jambe,
    - les points de liaison (6) sont disposés suivant des dessins et relient les couches étanches au liquide (3, 4) au matériau perméable à la vapeur des zones de séparation (7), au bord des bandes (14, 15),
    - lorsque la personne se lève, un liquide (1) situé dans les cavités (5), grâce à sa pression hydrostatique, peut exercer une action au moins partiellement compensatrice sur la pression artérielle qui règne dans les parties du corps couvertes.

2. Vêtement pour problèmes orthostatiques selon la revendication 1, caractérisé en ce qu'un réservoir (12) pour le liquide (1) est prévu et est situé sur la zone présentant les cavités (5).

3. Vêtement pour problèmes orthostatiques selon la revendication 1, caractérisé en ce que les zones de séparation (7) se composent d'un matériau perméable à la vapeur.

4. Vêtement pour problèmes orthostatiques selon l'une des revendications 1 à 3, caractérisé en ce que

    - les points de liaison (6) sont des segments droits qui sont sensiblement parallèles et dont la longueur correspond à peu près à leur écartement latéral,
    - les points de liaison (6) individuels sont situés sensiblement sur deux séries de lignes parallèles qui sont décalées suivant la moitié de l'écartement latéral de deux points de liaison (6) voisins, environ.

5. Vêtement pour problèmes orthostatiques selon l'une des revendications 1 à 3, caractérisé en ce que les zones présentant des cavités (5) sont disposées en bandes (14, 15) qui s'étendent sur toute la longueur dudit vêtement.

6. Vêtement pour problèmes orthostatiques selon la revendication 5, caractérisé en ce que les bandes (14) sont sensiblement droites.

7. Vêtement pour problèmes orthostatiques selon la revendication 5, caractérisé en ce que les bandes (15) sont en zigzag.

8. Vêtement pour problèmes orthostatiques selon l'une des revendications 1 à 7, caractérisé en ce qu'il comporte une partie supérieure en forme de culotte (26) qui couvre en supplément une partie au moins du tronc.

9. Vêtement pour problèmes orthostatiques selon la revendication 8, caractérisé en ce que

    - la partie supérieure en forme de culotte (26) couvre au moins la région abdominale,
    - ledit vêtement comporte une poche plate (13) qui est disposée dans la région abdominale, à l'intérieur dudit vêtement, et qui peut être reliée à celui-ci.

10. Vêtement pour problèmes orthostatiques selon la revendication 8, caractérisé en ce qu'il comporte une ceinture avec un réservoir (12) qui est relié audit vêtement.

11. Vêtement pour problèmes orthostatiques selon la revendication 8, caractérisé en ce que la partie supérieure en forme de culotte (26) a des bretelles (11).

12. Vêtement pour problèmes orthostatiques selon la revendication 11, caractérisé en ce qu'un réservoir (12) est ménagé dans les bretelles (11).

13. Vêtement pour problèmes orthostatiques selon l'une des revendications 1 à 12, caractérisé en ce qu'il comporte des pièces élastiques longitudinales.

14. Vêtement pour problèmes orthostatiques selon l'une des revendications 1 à 3, caractérisé en ce que les moyens de fermeture se composent d'une fermeture à glissière (21) pour chaque jambe.

15. Vêtement pour problèmes orthostatiques selon l'une des revendications 1 à 3, caractérisé en ce que les moyens d'adaptation se composent d'une fermeture autoagrippante (22, 23) pour chaque jambe, une partie (22) de la fermeture autoagrippante étant fixée à une patte (31) tandis que l'autre partie (23) est fixée à la partie dudit vêtement qui enveloppe le corps.

16. Vêtement pour problèmes orthostatiques selon les revendications 14 et 15, caractérisé en ce que les moyens de tension se composent de conduits (29) qui sont disposés le long du vêtement et dans la patte (31) entre la fermeture à glissière (21) et la fermeture autoagrippante (22), et qui peuvent être gonflés avec de l'air comprimé et produire ainsi une tension périphérique du vêtement correspondant à la pression minimale prévue.

17. Vêtement pour problèmes orthostatiques selon la revendication 1, caractérisé en ce qu'il est prévu, fixée à l'extrémité inférieure des bandes (14, 15), une patte (31) qui présente la même structure que les bandes (14, 15) et qui est remplie du même liquide que celles-ci, et en ce que les cavités (5) des bandes (14, 15) et de la patte (31) communiquent, ladite patte (31) pouvant être placée comme languette dans la chaussure de la personne qui porte le vêtement et être fixée sur le pied avec le dispositif de fermeture normal de la chaussure.

18. Vêtement pour problèmes orthostatiques selon la revendication 1, caractérisé en ce que les bandes (14, 15) peuvent être ramifiées.

Fig. 1

Fig. 2

**Fig. 3**

**Fig. 4**

**Fig. 5**

Fig. 8

Fig. 9

Fig. 7

Fig. 11

Fig. 10

Fig. 6